# EUROPEAN PATENT SPECIFICATION

(11) **EP 0 544 809 B1**
(45) Date of publication and mention of the grant of the patent: **16.12.1998**
(21) Application number: 91916483.0
(22) Date of filing: 20.08.1991
(51) Int. Cl.: C07H 21/00

(54) **METHODS OF SYNTHESIZING OLIGONUCLEOTIDES WITH RANDOM CODONS**
VERFAHREN ZUR HERSTELLUNG VON OLIGONUKLEOTIDEN MIT REGELLOSEN CODONEN
PROCEDE DE PRODUCTION PAR SYNTHESE D'OLIGONUCLEOTIDES AYANT DES CODONS ALEATOIRES

(30) Priority: 24.08.1990 US 573648
(43) Date of publication of application: 09.06.1993
(73) Proprietor: IXSYS, INC., San Diego, CA 92121 (US)
(72) Inventor: HUSE, William, D., Del Mar, CA 92014 (US)
(74) Representative: Goldin, Douglas Michael
(86) International application number: US9105939
(87) International publication number: WO9203461

(56) References cited:
- EP-A- 0 383 620
- US-A- 4 458 066
- NUCLEIC ACIDS RESEARCH vol. 11, no. 13 , 1983 , ARLINGTON, VIRGINIA US pages 4365 - 4377 R.FRANK ET. AL. 'A new general approach for the simultaneous chemical synthesis of large numbers of oligonucleotides: segmental solid supports'
- PROCEEDINGS OF THE NATIONAL ACADEMY OF SCIENCES OF USA vol. 82 , August 1985 , WASHINGTON US pages 5131 - 5135 R.A.HOUGHTEN 'General method for the rapid solid-phase synthesis of large numbers of peptides specificity of antigen-antibody interaction at the level of individual amino acids'
- R. ADAMS et al., "The Biochemistry of Nucleic Acids", published 1986 by Chapman and Hall (London/New York) see pages 11-13.
- Federation Proceedings, Volume 42, No. 7, issued 01 May 1983, BLAKE et al., "Tuplet Analysis of DNA sequences", page 2264, see Abstract 2958.

## Description

### BACKGROUND OF THE INVENTION

This invention relates generally to oligonucleotide synthesis and, more particularly, to methods of synthesizing oligonucleotides having random codons using individual monomers.

The speed and availability of automated nucleic acid synthesis has led to rapid technological advances in biological research. For example, the availability of synthetic primers for sequencing has permitted researchers to decrease their time and labor involved in sequencing a particular nucleic acid by approximately sixty percent. Another technology which is facilitated by synthetic oligonucleotides is the polymerase chain reaction (PCR). This technique, which involves the exponential amplification of sequences between two synthetic primers, offers unprecedented detection levels and permits genetic manipulation of the amplified sequence. Further, the availability of synthetic primers allows a variety of genetic manipulations to be performed with relatively simple procedures, including site-specific mutagenesis and the custom design of genetic vectors.

Sequences to be cloned are also routinely modified with synthetic oligonucleotides. The modifications of either vector or insert sequence can range from the addition of a simple sequence encoding a restriction enzyme site to more complicated schemes involving modifying the translation product of the cloned sequence with a specific peptide or a variety of peptide sequences. Thus, these technological advances associated with synthetic oligonucleotides has afforded researchers many opportunities to study diverse biological phenomenon in greater detail and with greater speed and accuracy.

Oligonucleotide synthesis proceeds via linear coupling of individual monomers in a stepwise reaction. The reactions are generally performed on a solid phase support by first coupling the 3' end of the first monomer to the support. The second monomer is added to the 5' end of the first monomer in a condensation reaction to yield a dinucleotide coupled to the solid support. At the end of each coupling reaction, the by-products and unreacted, free monomers are washed away so that the starting material for the next round of synthesis is the pure oligonucleotide attached to the support. In this reaction scheme, the stepwise addition of individual monomers to a single, growing end of a oligonucleotide ensures accurate synthesis of the desired sequence. Moreover, unwanted side reactions are eliminated, such as the condensation of two oligonucleotides, resulting in high product yields.

In some instances, it is desired that synthetic oligonucleotides have random nucleotide sequences. This result can be accomplished by adding equal proportions of all four nucleotides in the monomer coupling reactions, leading to the random incorporation of all nucleotides and yields a population of oligonucleotides with random sequences. Since all possible combinations of nucleotide sequences are represented within the population, all possible codon triplets will also be represented. If the objective is ultimately to generate random peptide products, this approach has a severe limitation because the random codons synthesized will bias the amino acids incorporated during translation of the DNA by the cell into polypeptides.

The bias is due to the redundancy of the genetic code. There are four nucleotide monomers which leads to sixty-four possible triplet codons. With only twenty amino acids to specify, many of the amino acids are encoded by multiple codons. Therefore, a population of oligonucleotides synthesized by sequential addition of monomers from a random population will not encode peptides whose amino acid sequence represents all possible combinations of the twenty different amino acids in equal proportions. That is, the frequency of amino acids incorporated into polypeptides will be biased toward those amino acids which are specified by multiple codons.

To alleviate amino acid bias due to the redundancy of the genetic code, the oligonucleotides can be synthesized from nucleotide triplets. Here, a triplet coding for each of the twenty amino acids is synthesized from individual monomers. Once synthesized, the triplets are used in the coupling reactions instead of individual monomers. By mixing equal proportions of the triplets, synthesis of oligonucleotides with random codons can be accomplished. However, the cost of synthesis from such triplets far exceeds that of synthesis from individual monomers because triplets are not commercially available.

Frank et al. Nucleic Acid research (1983) 11 (3) 4365-4377 is an example of a document which reaches a strategy for synthesis of a multiplicity of oligonucleotide sequences using monomers. An important feature of this strategy is performance of oligonucleotide synthesis on segmental solid supports, in particular cellulose disks. According to this strategy, groups of disks requiring coupling of the same monomer are grouped for an elongation reaction in the same reaction vessel. After each elongation reaction, the disks are regrouped ready for the next reaction. By this means, a mixture of oligonucleotides can be obtained with either random sequences or with certain predetermined sequences and a certain proportion of random sequences.

EP-A 0383260 describes the assembly of small oligonucleotide duplexes of multiples of three nucleotides to form longer oligonucleotide sequences encoding a mixture of polypeptides having predetermined amino acid constituents. Again, however, this strategy does not provide an answer to the problems set out above.

There thus exists a need for a method to synthesize oligonucleotides with random codons which alleviates genetic redundancy incurred through present synthesis methods using individual monomers and does not have the prohibitive costs associated with methods using pre-synthesized triplets. The present invention satisfies these needs and provides additional advantages as well.

### SUMMARY OF THE INVENTION

The invention provides a method of synthesizing oligonucleotides having random tuplets using individual monomers. The steps consist of: sequentially coupling monomers on separate supports to form at least two different tuplets, the coupling is performed in separate reaction vessels; mixing the supports from the reaction vessels; dividing the mixed supports into two or more separate reaction vessels; and repeating the coupling, mixing and dividing steps one or more times in the reaction vessels, ending with a mixing or dividing step. Additionally, the oligonucleotides can be cleaved from the supports.

### BRIEF DESCRIPTION OF THE DRAWINGS

Figure 1 is a schematic drawing for synthesizing oligonucleotides from nucleotide monomers with random tuplets at each position using twenty reaction vessels.

Figure 2 is a schematic drawing for synthesizing oligonucleotides from nucleotide monomers with random tuplets at each position using ten reaction vessels.

### DETAILED DESCRIPTION OF THE INVENTION

This invention is directed to a simple and inexpensive method for synthesizing oligonucleotides having random tuplets using individual monomers. The method is advantageous in that individual monomers are used instead of tuplets. The monomers are commercially available in a form which can be used in oligonucleotide synthesis and are inexpensive. An additional advantage of the method is that it can alleviate codon redundancy inherent in present methods of random synthesis by utilizing only a nondegenerate subset of all triplets. Thus, the method is able to produce a large proportion of possible oligonucleotides with random tuplets. The oligonucleotides produced are useful for making an unlimited number of pharmacological and research products.

In a preferred embodiment, the invention entails the sequential coupling of monomers to produce oligonucleotides with random codons. The coupling reactions for the randomization of twenty codons which specify the amino acids of the genetic code are performed in ten different reaction vessels. Each reaction vessel contains a support on which the monomers for two different codons are coupled in three sequential reactions. One of the reactions couple an equal mixture of two monomers such that the final product has a sequence of two different codons. The codons are randomized by removing the supports from the reaction vessels and mixing them to produce a single batch of supports containing all twenty codons. Synthesis at the next codon position proceeds by equally dividing the mixed batch of supports into ten reaction vessels as before and sequentially coupling the monomers for each pair of codons. The supports are again mixed to randomize the codons at the position just synthesized. The cycle of coupling, mixing and dividing continues until the desired number of codon positions have been randomized. After the last position has been randomized, the oligonucleotides with random codons are cleaved from the support.

As used herein, the term "monomer" or "nucleotide monomer" refers to individual nucleotides used in the chemical synthesis of oligonucleotides. Monomers that can be used include both the ribo- and deoxyribo- forms of each of the fivt standard nucleotides (derived from the bases adenine (A or dA, respectively), guanine (G or dG), cytosine (C or dC), thymine (T) and uracil (U)). Derivatives and precursors of bases such as inosine which are capable of supporting polypeptide biosynthesis are also included as monomers. Also included are chemically modified nucleotides, for example, one having a reversible blocking agent attached to any of the positions on the purine or pyrimidine bases, the ribose or deoxyribose sugar or the phosphate or hydroxyl moieties of the monomer. Such blocking groups include, for example, dimethoxytrityl, benzoyl, isobutyryl, beta-cyanoethyl and diisopropylamine groups, and are used to protect hydroxyls, exocyclic amines and phosphate moieties. Other blocking agents can also be used and are known to one skilled in the art.

As used herein, the term "tuplet" refers to a group of elements of a definable size. The elements of a tuplet as used herein are nucleotide monomers. For example, a tuplet can be a dinucleotide, a trinucleotide or can also be four or more nucleotides.

As used herein, the term "codon" or "triplet" refers to a tuplet consisting of three adjacent nucleotide monomers which specify one of the twenty naturally occurring amino acids found in polypeptide biosynthesis. The term also includes nonsense codons which do not specify any amino acid.

"Random codons" or "randomized codons," as used herein, refers to more than one codon at a position within a collection of oligonucleotides. The number of different codons can be from two to twenty at any particular position. "Randomized oligonucleotides," as used herein, refers to a collection of oligonucleotides with random codons at one or more positions. For example, if the randomized oligonucleotides are six nucleotides in length (i.e., two codons) and both the first and second codon positions are randomized to encode all twenty amino acids, then a population of oligonucleotides with every possible combination of the twenty triplets in the first and second position makes up the above population of randomized oligonucleotides. The number of possible codon combinations is 20². Likewise, if randomized oligonucleotides of fifteen nucleotides in length are synthesized which are randomized at all positions, then all triplets coding for each of the twenty amino acids will be found in equal proportions at every position. The population constituting the randomized oligonucleotides will contain 20¹⁵ different possible species of oligonucleotides. "Random tuplets," or "randomized tuplets" are defined analogously.

As used herein, the term "support" refers to a solid phase material for attaching monomers for chemical synthesis. Such support is usually composed of materials such as beads of control pore glass but can be other materials known to one skilled in the art. The term is also meant to include one or more monomers coupled to the support for additional oligonucleotide synthesis reactions.

As used herein, the terms "coupling" or "condensing" refers to the chemical reactions for attaching one monomer to a second monomer or to a solid support. Such reactions are known to one skilled in the art and are typically performed on an automated DNA synthesizer such as a MilliGen/Biosearch Cyclone Plus Synthesizer using procedures recommended by the manufacturer. "sequentially coupling" as used herein, refers to the stepwise addition of monomers.

The invention provides a method of synthesizing oligonucleotides having random tuplets using individual monomers. The method comprises several steps, the first being synthesis of a nucleotide tuplet for each tuplet to be randomized. As described here and below, a nucleotide triplet (i.e., a codon) will be used as a specific example of a tuplet. Any size tuplet will work using the methods disclosed herein, and one skilled in the art would know how to use the methods to randomize tuplets of any size.

If the randomization of codons specifying all twenty amino acids is desired at a position, then twenty different codons are synthesized. Likewise, if randomization of only ten codons at a particular position is desired then those ten codons are synthesized. Randomization of codons from two to sixty-four can be accomplished by synthesizing each desired triplet. Preferably, randomization of from two to twenty codons is used for any one position because of the redundancy of the genetic code. The codons selected at one position do not have to be the same codons selected at the next position. Additionally, the sense or anti-sense sequence oligonucleotide can be synthesized. The process therefore provides for randomization of any desired codon position with any number of codons.

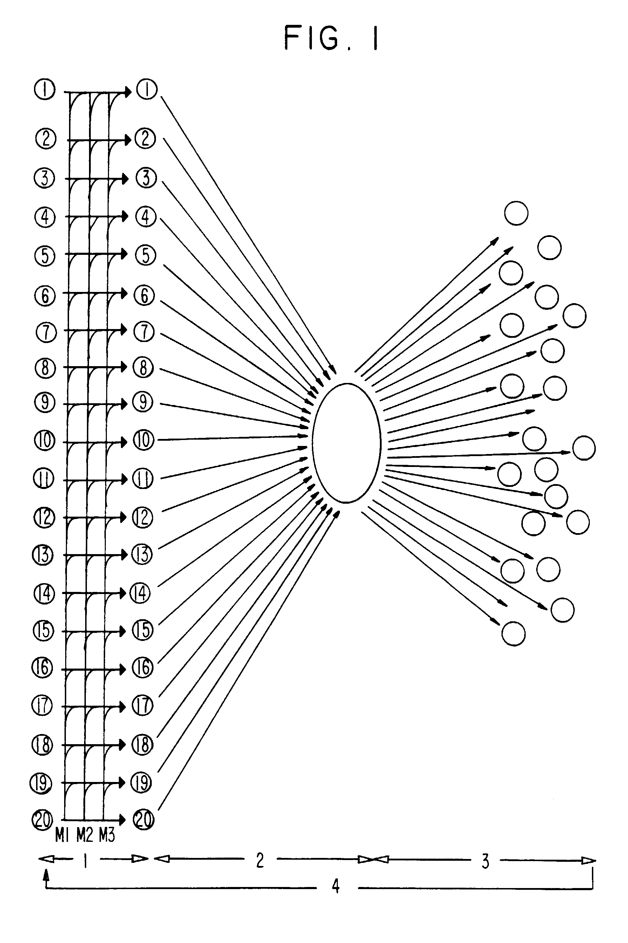

Codons to be randomized are synthesized sequentially by coupling the first monomer of each codon to separate supports. The supports for the synthesis of each codon can, for example, be contained in different reaction vessels such that one reaction vessel corresponds to the monomer coupling reactions for one codon. As will be used here and below, if twenty codons are to be randomized, then twenty reaction vessels can be used in independent coupling reactions for the first twenty monomers of each codon. Synthesis proceeds by sequentially coupling the second monomer of each codon to the first monomer to produce a dimer, followed by coupling the third monomer for each codon to each of the above-synthesized dimers to produce a trimer (Figure 1, step 1, where M₁, M₂ and M₃ represent the first, second and third monomer, respectively, for each codon to be randomized).

Following synthesis of the first codons from individual monomers, the randomization is achieved by mixing the supports from all twenty reaction vessels which contain the individual codons to be randomized. The solid phase support can be removed from its vessel and mixed to achieve a random distribution of all codon species within the population (Figure 1, step 2). The mixed population of supports, constituting all codon species, are then redistributed into twenty independent reaction vessels (Figure 1, step 3). The resultant vessels are all identical and contain equal portions of all twenty codons coupled to a solid phase support.

For randomization of the second position codon, synthesis of twenty additional codons is performed in each of the twenty reaction vessels produced in step 3 as the condensing substrates of step 1 (Figure 1, step 4). Steps 1 and 4 are therefore equivalent except that step 4 uses the supports produced by the previous synthesis cycle (steps 1 through 3) for codon synthesis whereas step 1 is the initial synthesis of the first codon in the oligonucleotide. The supports resulting from step 4 will each have two codons attached to them (i.e., a hexanucleotide) with the codon at the first position being any one of twenty possible codons (i.e., random) and the codon at the second position being one of the twenty possible codons.

For randomization of the codon at the second position and synthesis of the third position codon, steps 2 through 4 are again repeated. This process yields in each vessel a three codon oligonucleotide (i.e., 9 nucleotides) with codon positions 1 and 2 randomized and position three containing one of the twenty possible codons. Steps 2 through 4 are repeated to randomize the third position codon and synthesize the codon at the next position. The process is continued until an oligonucleotide of the desired length is achieved. After the final randomization step, the oligonucleotide can be cleaved from the supports and isolated by methods known to one skilled in the art. Alternatively, the oligonucleotides can remain on the supports for use in methods employing probe hybridization.

The diversity of codon sequences, i.e., the number of different possible oligonucleotides, which can be obtained using the methods of the present invention, is extremely large and only limited by the physical characteristics of available materials. For example, a support composed of beads of about 100 µm in diameter will be limited to about 10,000 beads/reaction vessel using a 1 µM reaction vessel containing 25 mg of beads. This size bead can support about 1 x 10⁷ oligonucleotides per bead. Synthesis using separate reaction vessels for each of the twenty amino acids will produce beads in which all the oligonucleotides attached to an individual bead are identical. The diversity which can be obtained under these conditions is approximately 10⁷ copies of 10,000 x 20 or 200,000 different random oligonucleotides. The diversity can be increased, however, in several ways without departing from the basic methods disclosed herein. For example, the number of possible sequences can be increased by decreasing the size of the individual beads which make up the support. A bead of about 30 µm in diameter will increase the number of beads per reaction vessel and therefore the number of oligonucleotides synthesized. Another way to increase the diversity of oligonucleotides with random codons is to increase the volume of the reaction vessel. For example, using the same size bead, a larger volume can contain a greater number of beads than a smaller vessel and therefore support the synthesis of a greater number of oligonucleotides. Increasing the number of codons coupled to a support in a single reaction vessel also increases the diversity of the random oligonucleotides. The total diversity will be the number of codons coupled per vessel raised to the number of codon positions synthesized. For example, using ten reaction vessels, each synthesizing two codons to randomize a total of twenty codons, the number of different oligonucleotides of ten codons in length per 100 µm bead can be increased where each bead will contain about 2¹⁰ or 1 x 10³ different sequences instead of one. One skilled in the art will know how to modify such parameters to increase the diversity of oligonucleotides with random codons.

The invention provides for a method of synthesizing oligonucleotides having random codons at each position using individual monomers wherein the number of reaction vessels is less than the number of codons to be randomized. For example, if twenty codons are to be randomized at each position within an oligonucleotide population, then ten reaction vessels can be used. The use of a smaller number of reaction vessels than the number of codons to be randomized at each position is preferred because the smaller number of reaction vessels is easier to manipulate and results in a greater number of possible oligonucleotides synthesized.

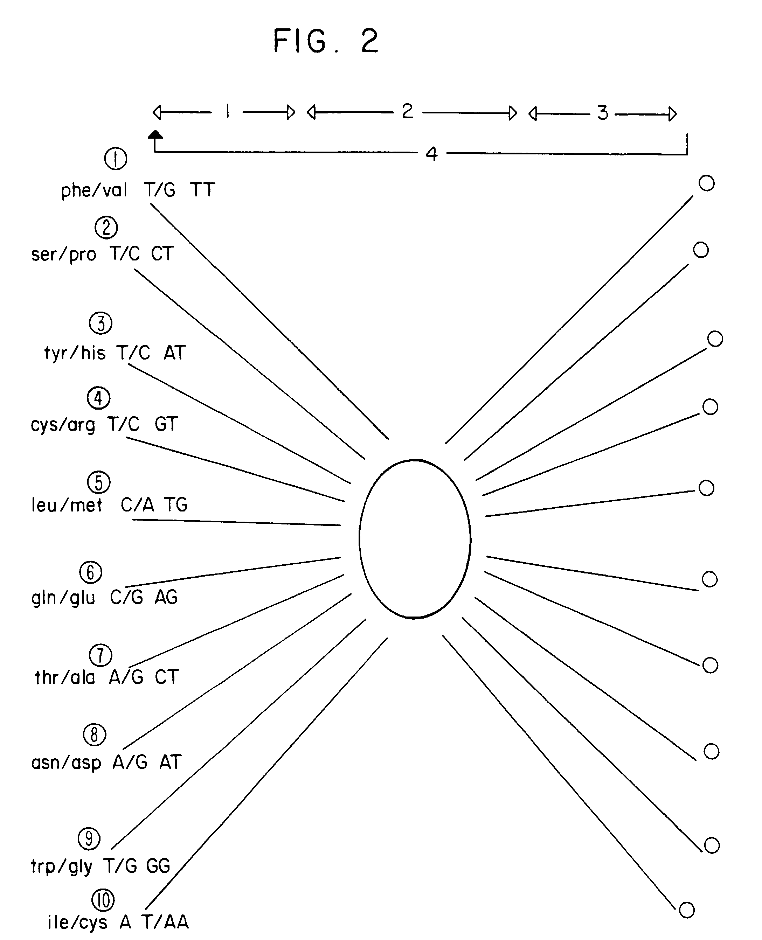

The use of a smaller number of reaction vessels for random synthesis of twenty codons at a desired position within an oligonucleotide is similar to that described above using twenty reaction vessels except that each reaction vessel can contain the synthesis products of more than one codon. For example, step one synthesis using ten reaction vessels proceeds by coupling about two different codons on supports contained in each of ten reaction vessels. This is shown in Figure 2 where each of the two codons coupled to a different support can consist of the following sequences: (1) (T/G)TT for Phe and Val; (2) (T/C)CT for Ser and Pro; (3) (T/C)AT for Tyr and His; (4) (T/C)GT for Cys and Arg; (5) (C/A)TG for Leu and Met; (6) (C/G)AG for Gln and Glu; (7) (A/G)CT for Thr and Ala; (8) (A/G)AT for Asn and Asp; (9) (T/G)GG for Trp and Gly and (10) A(T/A)A for Ile and Cys. The slash (/) signifies that a mixture of the monomers indicated on each side of the slash are used as if they were a single monomer in the indicated coupling step. The antisense sequence for each of the above codons can be generated by synthesizing the complementary sequence. For example, the antisense for Phe and Val can be AA(C/A). The amino acids encoded by each of the above pairs of sequences are given as the standard three letter nomenclature.

Coupling of the monomers in this fashion will yield codons specifying all twenty of the naturally occurring amino acids attached to supports in ten reaction vessels. However, the number of individual reaction vessels to be used will depend on the number of codons to be randomized at the desired position and can be determined by one skilled in the art. For example, if ten codons are to be randomized, then five reaction vessels can be used for coupling. The codon sequences given above can be used for this synthesis as well. The sequences of the codons can also be changed to incorporate or be replaced by any of the additional forty-four codons which constitutes the genetic code.

The remaining steps of synthesis of oligonucleotides with random codons using a smaller number of reaction vessels are as outlined above for synthesis with twenty reaction vessels except that the mixing and dividing steps are performed with supports from about half the number of reaction vessels. These remaining steps are shown in Figure 2 (steps 2 through 4).

The invention also provides a method of synthesizing oligonucleotides using individual monomers having at least one specified tuplet at a predetermined position and the remaining positions having random tuplets. The synthesis steps are similar to those outlined above using twenty or less reaction vessels except that prior to synthesis of the specified codon position, the dividing of the supports into separate reaction vessels for synthesis of different codons is omitted. For example, if the codon at the second position of the oligonucleotide is to be specified, then following synthesis of random codons at the first position and mixing of the supports, the mixed supports are not divided into new reaction vessels but, instead, can be contained in a single reaction vessel to synthesize the specified codon. The specified codon is synthesized sequentially from individual monomers as described above. Thus, the number of reaction vessels can be increased or decreased at each step to allow for the synthesis of a specified codon or a desired number of random codons.

Following codon synthesis, the mixed supports are divided into individual reaction vessels for synthesis of the next codon to be randomized (Figure 1, step 3) or can be used without separation for synthesis of a consecutive specified codon. The rounds of synthesis can be repeated for each codon to be added until the desired number of positions with predetermined or randomized codons are obtained.

Synthesis of oligonucleotides with the first position codon being specified can also be synthesized using the above method. In this case, the first position codon is synthesized from the appropriate monomers. The supports are divided into the required number of reaction vessels needed for synthesis of random codons at the second position and the rounds of synthesis, mixing and dividing are performed as described above.

The invention provides for a method of synthesizing oligonucleotides having tuplets which are diverse but biased toward a predetermined sequence. This method employs two reaction vessels, one vessel for the synthesis of a predetermined sequence and the second vessel for the synthesis of a random sequence. The method is advantageous to use when a significant number of codon positions, for example, are to be of a specified sequence since it alleviates the use of multiple reaction vessels. Instead, a mixture of four different monomers such as adenine, guanine, cytosine and thymine nucleotides are used for the first and second monomers in the codon position specifying the diverse sequence. The codon is completed by coupling a mixture of a pair of monomers of either guanine and thymine or cytosine and adenine nucleotides at the third monomer position. In the second vessel, nucleotide monomers are coupled sequentially to yield the predetermined codon sequence. Mixing of the two supports yields a population of oligonucleotides containing both the predetermined codon and the random codons at the desired position. Synthesis can proceed by using this mixture of supports in a single reaction vessel, for example, for coupling additional predetermined codons or, further dividing the mixture into two reaction vessels for synthesis of additional random codons.

The two reaction vessel method can be used for codon synthesis within an oligonucleotide with a predetermined tuplet sequence by dividing the support mixture into two portions at the desired codon position to be randomized. Additionally, this method allows for the extent of randomization to be adjusted. For example, unequal mixing or dividing of the two supports will change the fraction of codons with predetermined sequences compared to those with random codons at the desired position. Unequal mixing and dividing of supports can be useful when there is a need to synthesize random codons at a significant number of positions within an oligonucleotide of a longer or shorter length.

The extent of randomization can also be adjusted by using unequal mixtures of monomers in the first, second and third monomer coupling steps of the random codon position. The unequal mixtures can be in any or all of the coupling steps to yield a population of codons enriched in sequences reflective of the monomer proportions.

The invention provides for oligonucleotides synthesized by the methods described herein. Synthesis of randomized oligonucleotides is performed using methods well known to one skilled in the art. Linear coupling of monomers can, for example, be accomplished using phosphoramidite chemistry with a MilliGen/Biosearch Cyclone Plus automated synthesizer as described by the manufacturer (Millipore, Burlington, MA). Other chemistries and automated synthesizers can be employed as well and are known to one skilled in the art.

Synthesis of multiple codons can be performed without modification to the synthesizer by separately synthesizing the codons in individual sets of reactions. Alternatively, modification of an automated DNA synthesizer can be performed for the simultaneous synthesis of codons in multiple reaction vessels.

The following examples are intended to illustrate, but not limit the invention.

### EXAMPLE I

### Synthesis of an Oligonucleotide with Random Codons Using Individual Monomers

This example demonstrates the synthesis of an antisense oligonucleotide of ten codons in length with twenty random codons at each position.

The reaction vessels were obtained from the manufacturer of the automated synthesizer (Millipore, Burlington, MA; supplier of MilliGen/Biosearch Cyclone Plus Synthesizer) and were obtained as packages consisting of empty reaction columns (1 µmole), frits, crimps and plugs (MilliGen/Biosearch catalog # GEN 860458). Derivatized and underivatized control pore glass phosphoramidite nucleotides, and synthesis reagents were also obtained from MilliGen/Biosearch. Crimper and decrimper tools were obtained from Fisher Scientific Co., Pittsburgh, PA (Catalog numbers 06-406-20 and 06-406-25A, respectively).

Ten reaction columns are used for the synthesis of a ten codon random oligonucleotide. The oligonucleotide has 5 monomers at its 3' end of the sequence 5'GAGCT3' and 8 monomers at its 5' end of the sequence 5'AATTCCAT3'. The synthesizer is fitted with a column derivatized with a thymine nucleotide (MilliGen/Biosearch # 0615.50) and is programmed to synthesize the sequences shown in Table I for each of ten columns in independent reaction sets. The sequence of the last three monomers (from right to left since synthesis proceeds 3' to 5') encode the indicated amino acids:

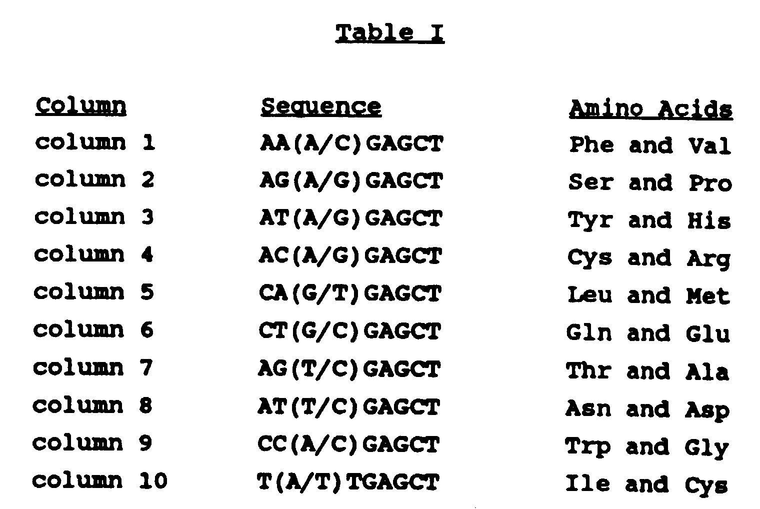

where the two monomers in parentheses denote a single monomer position within the codon and indicate that an equal mixture of each monomer is added to the reaction for coupling. The monomer coupling reactions for each of the 10 columns are performed as recommended by the manufacturer (amidite version S1.06, # 8400-050990, scale 1 µM). After the last coupling reaction, the columns are washed with acetonitrile and lyophilized to dryness.

Following synthesis, the plugs are removed from each column using a decrimper and the reaction products are poured into a single weigh boat. Lost material is equalized with underivatized control pore glass and mixed thoroughly to obtain a random distribution of all twenty codon species. The reaction products are then aliquoted into 10 new reaction columns by removing 25 mg of material at a time and placing it into separate reaction columns. Alternatively, the reaction products can be aliquoted by suspending the beads in a liquid that is dense enough for the beads to remain dispersed, preferably a liquid that is equal in density to the beads, and then aliquoting equal volumes of the suspension into separate reaction columns. The lip on the inside of the columns where the frits rest are cleared of material using vacuum suction with a syringe and 25 G needle. New frits are placed onto the lips, the plugs are fitted into the columns and are crimped into place using a crimper.

Synthesis of the second codon position is achieved using the above 10 columns containing the random mixture of reaction products from the first codon synthesis. The monomer coupling reactions for the second codon position are shown in Table II. An A in the first position means that any monomer can be programmed into the synthesizer at that position since the first monomer position is not coupled by the synthesizer. An A also denotes that the columns from the previous codon synthesis should be placed on the synthesizer for use in the present synthesis round. Reactions are again sequentially repeated for each column as shown in Table II and described above, and the reaction products washed and dried.

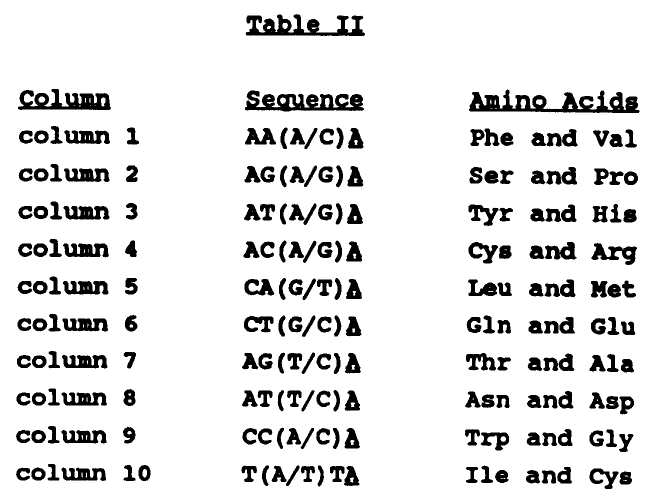

Randomization of the second codon position is achieved by removing the reaction products from each of the columns and thoroughly mixing the material. The material is again divided into new reaction columns and prepared for monomer coupling reactions as described above.

Random synthesis of the next seven codons (positions 3 through 9) proceeds identically to the cycle described above for the second codon position and again used the monomer sequences of Table II. Each of the newly repacked columns containing the random mixture of reaction products from synthesis of the previous codon position is used for the synthesis of the subsequent codon position. After synthesis of the codon at position nine and mixing of the reaction products, the material is divided and repacked into 40 different columns and the monomer sequences shown in Table III are coupled to each of the 40 columns in independent reactions. The oligonucleotides from each of the 40 columns are mixed once more and cleaved from the control pore glass as recommended by the manufacturer.

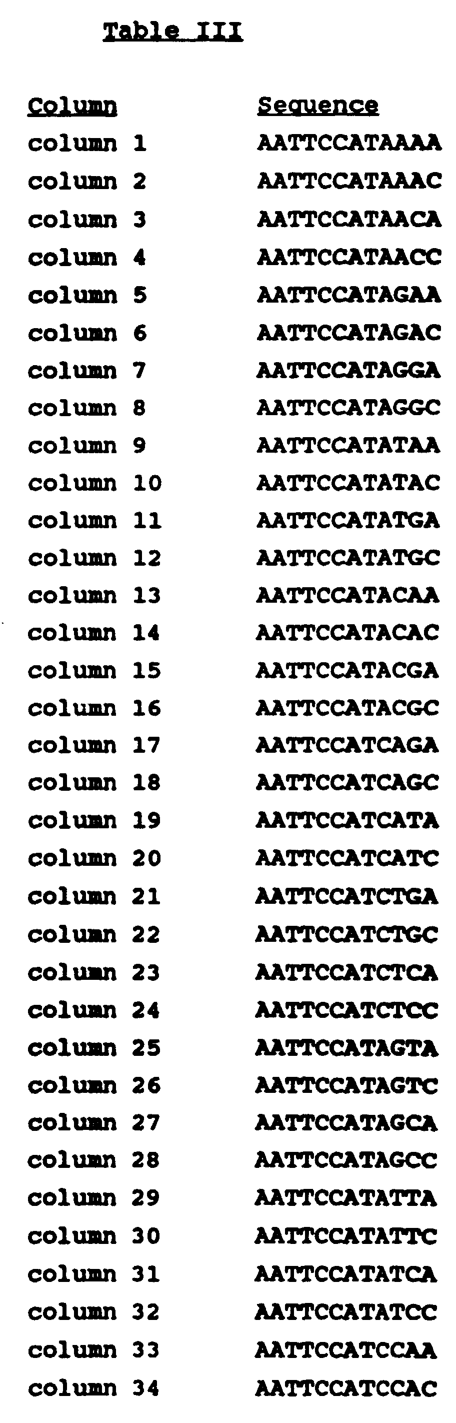

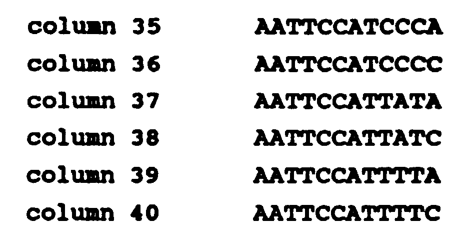

### EXAMPLE II

### Synthesis of a Randomized Oligonucleotides with Predetermined Positions Having Specified Codons

This example demonstrates the synthesis of a random oligonucleotide with the second position specifying the codon for methionine.

Synthesis of a random oligonucleotide having a specified codon at the second position is accomplished identically to that described in Example I except that the monomer sequence synthesized at the second position is identical for all ten reaction columns. The reaction columns containing the random codons, after the first position synthesis, are placed on the synthesizer and all ten columns receive the identical sequence of monomer coupling reactions. For methionine being the specified codon at the second position, the sequence is ATG. After all the coupling reactions are performed, the resultant product is a dinucleotide with the first position being random and the second position having a methionine codon. The columns are then used for the ten coupling reactions, as described in Example I to synthesize random codons at the remaining positions.

### EXAMPLE III

### Synthesis of Oligonucleotides Having Triplets which are Diverse but Biased Toward a Predetermined Sequence

This example demonstrates the use of the two column method for synthesis of a thirteen codon oligonucleotide in which five positions are diverse but biased toward a predetermined sequence.

Synthesis of the thirteen codon oligonucleotide was accomplished using the methods described in Example I. As an alternative, smaller beads having a capacity of 48 µg/g (Genta, San Diego, CA) can be used for the synthesis. Such beads are derivatized with a guanine nucleotide and do not have a controlled pore size. The first four codon positions as well as the last four positions of the oligonucleotide were synthesized to contain predetermined codon sequences. The middle five codon positions, however, were synthesized to contain a diverse sequence that was biased toward a predetermined sequence. The overall scheme can be depicted as the synthesis of the following two oligonucleotides where oligonucleotide (1) shows the sequence of the predetermined sequence and oligonucleotide (2) shows the positions of the five randomized codon positions:

(1) 5'- GTA CCA GTT CAT GAA ACT TAC
ACT TCA GCT GGC CCT GCA - 3'

(2) 5'- GTA CCA GTT CAT C/ANN C/ANN
C/ANN C/ANN C/ANN GCT GGC CCT GCA - 3'

The synthesis of the first four codon positions was accomplished by the sequential coupling of monomers in a single reaction column (5'- GCT GGC CCT GCA -3'). Since oligonucleotide synthesis proceeds in a 3'-to-5' direction, these first four synthesized positions correspond to the last four codon positions of the desired oligonucleotide. After coupling of the last monomer, the column was unplugged and its contents divided into two equal portions as described previously. Each portion was then repacked into two empty reaction columns. Reaction columns were plugged as described previously and the columns were placed on the synthesizer and programmed to synthesize the following sequences.

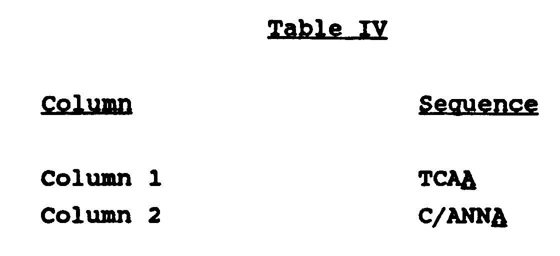

Following synthesis the plugs were removed and the products were mixed, divided into two equal portions and then repacked into new columns as described in Example I. Synthesis, mixing and dividing of the next four positions proceeded identical to that described above for the fifth synthesized position. The sequences coupled at each position for each of the two columns are shown below in Table V.

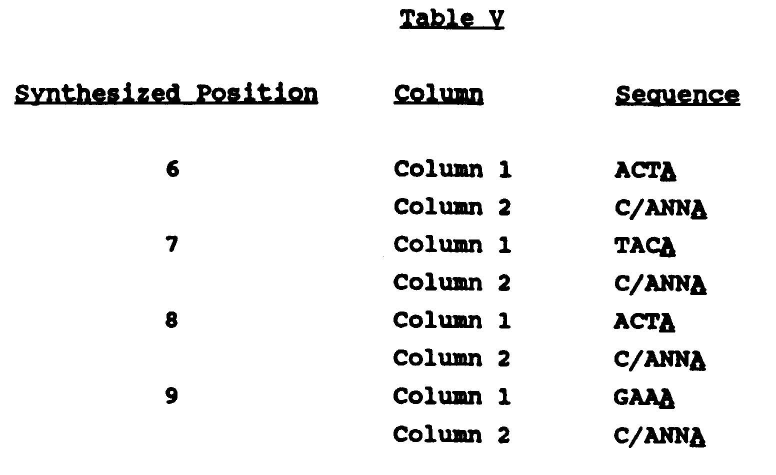

Synthesized positions 10 through 13 were performed using a single reaction column that contained as the starting material the combined products of columns 1 and 2 after position 9 was synthesized. These last four positions were synthesized by the sequential coupling of individual monomers to yield a population of oligonucleotides in which the first four and the last four codon positions were of a predetermined sequence, and the middle five positions were diverse but biased toward a predetermined sequence.

The population of oligonucleotides synthesized above were cleaved and purified from the beads. Sequences of twenty-four randomly selected oligonucleotides were determined by DNA sequencing after their incorporation by mutagenesis into a M13-derived vector. Nine of these sequences contained the vector sequence and one contained a 1 bp deletion due to the synthesis or mutagenesis procedures. These ten oligonucleotide sequences were not incorporated into the analysis shown below. All of the remaining 14 oligonucleotide sequences had a codon different than that specified by the predetermined sequence incorporated at at least one position. Many of the oligonucleotide sequences had different codons at more than one position. (Two oligonucleotide sequences had only one position changed whereas four oligonucleotides had each of two, three or four different positions changed.) There were no oligonucleotide sequences found that contained different codons at all five positions. However, in subsequent analysis of further selected clones, oligonucleotide sequences were found that contained different codons at all five positions. The frequency of different codons obtained at randomized positions 5 through 9 of the thirteen codon oligonucleotides is shown below in Table VI.

**Table VI**

| | NUMBER OBTAINED | |
|---|---|---|
| Codon Position | Predetermined Codon Sequences | Random Codon Sequences |
| 5 | 9 | 5 |
| 6 | 5 | 9 |
| 7 | 5 | 9 |
| 8 | 9 | 5 |
| 9 | 4 | 10 |

These results demonstrate that oligonucleotides can be synthesized using the two-column method described herein to obtain a population of oligonucleotides biased toward a predetermined sequence but having diverse codon sequences at one or more positions.

### EXAMPLE IV

### The Use of liquid Suspensions for Mixing and Dividing Reaction Products

This example shows the use of liquid suspensions for the aliquoting of equal volumes of reaction products at each mixing and dividing step.

The mixing and dividing steps described in Example I for generating random distributions of reaction products have been performed by the alternative method of dispensing equal volumes of bead suspensions. The liquid chosen that is dense enough for the beads to remain dispersed was 100% acetonitrile.

Briefly, each column was prepared for the first coupling reaction by suspending 22 mg (1µmole) of 48 µg/g capacity beads (Genta, San Diego, CA) in 0.5 mls of 100% acetonitrile and transferring this suspension to an empty reaction column. The beads were kept relatively dispersed by gently pipetting the suspension during transfer. Columns were plugged and monomer coupling reactions were performed for each desired codon. After coupling of the last monomer for each desired codon, the columns were unplugged as described previously and their contents were poured into a 50 ml conical centrifuge tube. The columns were rinsed with 100% acetonitrile to recover any remaining beads. The volume used for rinsing was determined so that the final volume of total bead suspension was about 0.5 mls for each new reaction column that the beads would be aliquoted into. The mixture was vortexed gently to produce a uniformly dispersed suspension and then divided, with constant pipetting of the mixture, into equal volumes. Each mixture of beads was then transferred to an empty reaction column. The empty tubes were washed with a small volume of 100% acetonitrile and also transferred to their respective columns.

Although the invention has been described with reference to the presently preferred embodiment, it should be understood that various modifications can be made without departing from the spirit of the invention. Accordingly, the invention is limited only by the claims.

## Claims

1. A method of synthesizing oligonucleotides having random tuplets using individual monomers comprising the steps of:
(1) sequentially coupling monomers on separate supports to form at least two different tuplets, said coupling being performed in separate reaction vessels;
(2) mixing the supports from the separate reaction vessels;
(3) dividing the mixed supports into two or more separate reaction vessels; and
(4) repeating steps (1) through (3) one or more times in the reaction vessels of step (3), wherein the last step ends at step (2).

2. The method of claim 1 further comprising step (5) cleaving said oligonucleotides with random tuplets from said support.

3. The method of claim 1, wherein the tuplet is a triplet.

4. The method of claim 1, wherein the number of tuplets to be randomized is between two and twenty.

5. The method of claim 1, wherein the number of reaction vessels is equal to the number of tuplets to be randomized.

6. The method of claim 5, wherein each tuplet to be randomized is coupled in a separate reaction vessel.

7. The method of claim 1, wherein the number of reaction vessels is less than the number of tuplets to be randomized.

8. The method of claim 7, wherein two different tuplets to be randomized are coupled to a support in a single reaction vessel.

9. The method of claim 7, wherein the first, second and third monomers of the tuplet are selected from (T/G)TT, (T/C)CT, (T/C)AT, (T/C)GT, (C/A)TG, (C/G)AG, (A/G)CT, (A/G)AT, (T/G)GG or A(T/A)A.

10. A method of synthesizing oligonucleotides using individual monomers having at least one specified tuplet at a predetermined position and the remaining positions having random tuplets comprising the method of claim 1, wherein said specified tuplet position is synthesized on the mixed supports prior to dividing into separate reaction vessels.

11. The method of claim 10, wherein the first position is predetermined and the specified tuplet is synthesized prior to step (1).

12. A method of synthesizing oligonucleotides having tuplets which are diverse but biased toward a predetermined sequence comprising the steps of:
(1) sequentially coupling monomers on a support to form a tuplet with a predetermined sequence, said coupling being performed in a first reaction vessel;
(2) sequentially coupling monomers on a support to form a random tuplet, said coupling being performed in a second reaction vessel;
(3) mixing the supports from said first and second reaction vessels; and
(4) dividing the mixed supports from step (3) and using the supports in step (1) and step (2), wherein steps (1) through (3) are repeated at least once.

13. The method of claim 12 further comprising step (4) cleaving said oligonucleotide from said support.

14. The method of claim 12, wherein said tuplet is a triplet and step (2) further comprises:
(2a) coupling a mixture of four different monomers for the first monomer of the random triplet;
(2b) coupling a mixture of four different monomers for the second monomer of the random triplet; and
(2c) coupling a pair of monomers selected from the group consisting of guanine and thymine or cytosine and adenine monomers for the third monomer of the random triplet to form a random triplet.

15. The method of claim 14, wherein the four different monomers are adenine, guanine, cytosine and thymine.

16. The method of claim 14, wherein four different monomers in step (2a) or (2b) are present in equal proportions.

17. The method of claim 14, wherein the four different monomers in steps (2a) and (2b) are present in equal proportions.

18. The method of claim 14, wherein the four different monomers in steps (2a) or (2b) are present in unequal proportions.

19. The method of claim 14, wherein the four different monomers in steps (2a) and (2b) are present in unequal proportions.

20. The method of claim 14, wherein the guanine and thymine monomers of step (2c) are present in equal proportions.

21. The method of claim 14, wherein the guanine and thymine monomers of step (2c) are present in unequal proportions.

22. The method of claim 12, wherein the mixed supports from said first and second reaction vessels are divided into equal proportions.

23. The method of claim 12, wherein the mixed supports from said first and second reaction vessels are divided into unequal proportions.

24. A diverse population of oligonucleotides synthesized by the method of claim 1.

25. A diverse population of oligonucleotides synthesized by the method of claim 12.

## Patentansprüche

1. Verfahren zum Synthetisieren von Oligonukleotiden mit Zufallstupletts unter Verwendung individueller Monomere, umfassend die folgenden Schritte:
(1) sequenzielles Koppeln von Monomeren auf getrennten Trägern, um mindestens zwei verschiedene Tupletts zu bilden, wobei die Kopplung in getrennten Reaktionsgefäßen durchgeführt wird;
(2) Mischen der Träger aus den getrennten Reaktionsgefaßen;
(3) Aufteilen der gemischten Träger in zwei oder mehr getrennte Reaktionsgefäße; und
(4) ein- oder mehrmaliges Wiederholen der Schritte (1) bis (3) in den Reaktionsgefaßen von Schritt (3), wobei der letzte Schritt bei Schritt (2) endet.

2. Verfahren von Anspruch 1, ferner umfassend den Schritt (5) des Abspaltens der Oligonukleotide mit Zufallstupletts von dem Träger.

3. Verfahren von Anspruch 1, wobei das Tuplett ein Triplett ist.

4. Verfahren von Anspruch 1, wobei die Anzahl von Tupletts, die zufallsangeordnet werden sollen, zwischen zwei und zwanzig liegt.

5. Verfahren von Anspruch 1, wobei die Anzahl von Reaktionsgefäßen gleich der Anzahl von Tupletts, die zufallsangeordnet werden sollen, ist.

6. Verfahren von Anspruch 5, wobei jedes Tuplett, das zufallsangeordnet werden soll, in einem separaten Reaktionsgefäß gekoppelt wird.

7. Verfahren von Anspruch 1, wobei die Anzahl von Reaktionsgefäßen geringer als die Anzahl von zufällig anzuordnenden Tupletts ist.

8. Verfahren von Anspruch 7, wobei zwei verschiedene, zufällig anzuordnende Tupletts an einen Träger in einem einzelnen Reaktionsgefäß gekoppelt werden.

9. Verfahren von Anspruch 7, wobei die ersten, zweiten und dritten Monomeren des Tupletts gewählt werden aus (T/G)TT, (T/C)CT, (T/C)AT; (T/C)GT, (C/A)TG, (C/G)AG, (A/G)CT, (A/G)AT, (T/G)GG oder A(T/A)A.

10. Verfahren zur Synthese von Oligonukleotiden unter Verwendung individueller Monomere mit mindestens einem spezifisch angegebenen Tuplett an einer vorbestimmten Position und den restlichen Positionen mit Zufallstupletts, umfassend das Verfahren von Anspruch 1, wobei die spezifisch angegebene Tuplettposition auf den gemischten Trägern vor Aufteilung in getrennte Reaktionsgefäße synthetisiert wird.

11. Verfahren von Anspruch 10, wobei die erste Position vorbestimmt ist und das spezifisch angegebene Tuplett vor Schritt (1) synthetisiert wird.

12. Verfahren zur Synthese von Oligonukleotiden mit Tupletts, welche verschiedenartig sind, aber zu einer vorbestimmten Sequenz neigen, umfassend die folgenden Schritte:
(1) sequenzielles Koppeln von Monomeren auf einem Träger, um ein Tuplett mit einer vorbestimmten Sequenz zu bilden, wobei die Kopplung in einem ersten Reaktionsgefäß durchgeführt wird;
(2) sequenzielles Koppeln von Monomeren auf einem Träger, um ein Zufallstuplett zu bilden, wobei die Kopplung in einem zweiten Reaktionsgefäß durchgeführt wird;
(3) Mischen der Träger aus den ersten und zweiten Reaktionsgefaßen; und
(4) Aufteilen der gemischten Träger aus Schritt (3) und Verwenden der Träger in Schritt (1) und Schritt (2), wobei die Schritte (1) bis (3) mindestens einmal wiederholt werden.

13. Verfahren von Anspruch 12, ferner umfassend den Schritt (4) des Abspaltens des Oligonukleotides von dem Träger.

14. Verfahren von Anspruch 12, wobei es sich bei dem Tuplett um ein Triplett handelt und der Schritt (2) ferner umfaßt:
(2a) Koppeln einer Mischung von vier verschiedenen Monomeren für das erste Monomer des Zufallstripletts;
(2b) Koppeln einer Mischung von vier verschiedenen Monomeren für das zweite Monomer des Zufallstripletts; und
(2c) Koppeln eines Paares von Monomeren, gewählt aus der Gruppe, bestehend aus Guanin- und Thymin- oder Cytosin- und Adeninmonomeren, für das dritte Monomer des Zufallstripletts, um ein Zufallstriplett zu bilden.

15. Verfahren von Anspruch 14, wobei die vier verschiedenen Monomere Adenin, Guanin, Cytosin und Thymin sind.

16. Verfahren von Anspruch 14, wobei vier verschiedene Monomere in Schritt (2a) oder (2b) in gleichen Anteilen vorhanden sind.

17. Verfahren von Anspruch 14, wobei die vier verschiedenen Monomere in den Schritten (2a) und (2b) in gleichen Anteilen vorhanden sind.

18. Verfahren von Anspruch 14, wobei die vier verschiedenen Monomere in den Schritten (2a) oder (2b) in ungleichen Anteilen vorhanden sind.

19. Verfahren von Anspruch 14, wobei die vier verschiedenen Monomere in den Schritten (2a) und (2b) in ungleichen Anteilen vorhanden sind.

20. Verfahren von Anspruch 14, wobei die Guanin- und Thyminmonomere von Schritt (2c) in gleichen Anteilen vorhanden sind.

21. Verfahren von Anspruch 14, wobei die Guanin- und Thyminmonomere von Schritt (2c) in ungleichen Anteilen vorhanden sind.

22. Verfahren von Anspruch 12, wobei die gemischten Träger aus den ersten und zweiten Reaktionsgefäßen zu gleichen Anteilen aufgeteilt werden.

23. Verfahren von Anspruch 12, wobei die gemischten Träger aus den ersten und zweiten Reaktionsgefäßen zu ungleichen Anteilen aufgeteilt werden.

24. Verschiedenartige Population von Oligonukleotiden, synthetisiert durch das Verfahren von Anspruch 1.

25. Verschiedenartige Population von Oligonukleotiden, synthetisiert durch das Verfahren von Anspruch 12.

## Revendications

1. Une méthode de synthèse d'oligonucléotides ayant des tuplets aléatoires utilisant des monomères individuels comprenant les étapes consistant à :
(1) coupler séquentiellement des monomères sur des supports séparés pour former au moins deux tuplets différents, ledit couplage étant réalisé dans des récipients réactionnels séparés ;
(2) mélanger les supports des récipients réactionnels séparés ;
(3) répartir les supports mélangés dans deux récipients réactionnels ou plus ; et
(4) répéter les étapes (1) à (3) une ou plusieurs fois dans les récipients réactionnels de l'étape (3), où la dernière étape se termine à l'étape (2).

2. La méthode de la revendication 1 comprenant en outre l'étape (5) de clivage desdits oligonucléotides avec des tuplets aléatoires à partir dudit support.

3. La méthode de la revendication 1, dans laquelle le tuplet est un triplet.

4. La méthode de la revendication 1, dans laquelle le nombre de tuplets à randomiser est compris entre deux et vingt.

5. La méthode de la revendication 1, dans laquelle le nombre de récipients réactionnels est égal au nombre de tuplets à randomiser.

6. La méthode de la revendication 5, dans laquelle chaque tuplet à randomiser est couplé dans un récipient réactionnel séparé.

7. La méthode de la revendication 1, dans laquelle le nombre de récipients réactionnels est inférieur au nombre de tuplets à randomiser.

8. La méthode de la revendication 7, dans laquelle deux tuplets différents à randomiser sont couplés à un support dans un seul récipient réactionnel.

9. La méthode de la revendication 7, dans laquelle les premier, deuxième et troisième monomères du tuplet sont sélectionnés à partir de (T/G)TT, (T/C)CT, (T/C)AT, (T/C)GT, (C/A)TG, (C/G)AG, (A/G)CT, (A/G)AT, (T/G)GG ou A(T/A)A.

10. Une méthode de synthèse d'oligonucléotides utilisant des monomères individuels ayant au moins un tuplet spécifié à une position prédéterminée et les positions restantes ayant des tuplets aléatoires comprenant la méthode de la revendication 1, dans laquelle ladite position du tuplet spécifié est synthétisée sur les supports mélangés avant de les répartir dans des récipients réactionnels séparés.

11. La méthode de la revendication 10, dans laquelle la première position est prédéterminée et le tuplet spécifié est synthétisé avant l'étape (1).

12. Une méthode de synthèse d'oligonucléotides ayant des tuplets qui sont variés mais biaisés vers une séquence prédéterminée comprenant les étapes consistant à :
(1) coupler séquentiellement des monomères sur un support pour former un tuplet de séquence prédéterminée, ledit couplage étant réalisé dans un premier récipient réactionnel ;
(2) coupler séquentiellement des monomères sur un support pour former un tuplet aléatoire, ledit couplage étant réalisé dans un second récipient réactionnel ;
(3) mélanger les supports desdits premier et second récipients réactionnels ; et
(4) répartir les supports mélangés de l'étape (3) et en utilisant les supports à l'étape (1) et à l'étape (2), où les étapes (1) à (3) sont répétées au moins une fois.

13. La méthode de la revendication 12 comprenant en outre l'étape (4)de clivage dudit oligonucléotide à partir dudit support.

14. La méthode de la revendication 12, dans laquelle ledit tuplet est un triplet et l'étape (2) comprend en outre :
(2a) le couplage d'un mélange de quatre monomères différents pour le premier monomère du triplet aléatoire ;
(2b) le couplage d'un mélange de quatre monomères différents pour le second monomère du triplet aléatoire ; et
(2c) le couplage d'une paire de monomères choisis dans le groupe constitué de monomères de guanine et de thymine ou de cytosine et d'adénine pour le troisième monomère du triplet aléatoire afin de former un triplet aléatoire.

15. La méthode de la revendication 14, dans laquelle les quatre monomères différents sont l'adénine, la guanine, la cytosine et la thymine.

16. La méthode de la revendication 14, dans laquelle les quatre monomères différents de l'étape (2a) ou (2b) sont présents en proportions égales.

17. La méthode de la revendication 14, dans laquelle les quatre monomères différents des étapes (2a) et (2b) sont présents en proportions égales.

18. La méthode de la revendication 14, dans laquelle les quatre monomères différents des étapes (2a) ou (2b) sont présents en proportions inégales.

19. La méthode de la revendication 14, dans laquelle les quatre monomères différents des étapes (2a) et (2b) sont présents en proportions inégales.

20. La méthode de la revendication 14, dans laquelle les monomères de guanine et de thymine de l'étape (2c) sont présents en proportions égales.

21. La méthode de la revendication 14, dans laquelle les monomères de guanine et de thymine de l'étape (2c) sont présents en proportions inégales.

22. La méthode de la revendication 12, dans laquelle les supports mélangés desdits premier et second récipients réactionnels sont répartis en proportions égales.

23. La méthode de la revendication 12, dans laquelle les supports mélangés desdits premier et second récipients réactionnels sont répartis en proportions inégales.

24. Une population diverse d'oligonucléotides synthétisés par la méthode de la revendication 1.

25. Une population diverse d'oligonucléotides synthétisés par la méthode de la revendication 12.
